# EUROPEAN PATENT APPLICATION

(11) **EP 2 444 112 A1**
(43) Date of publication of application: **25.04.2012**
(21) Application number: 10788864.6
(22) Date of filing: 07.06.2010
(51) Int. Cl.: A61M 11/00

(54) **HIGH-FREQUENCY INDUCTION ATOMIZATION DEVICE**

(30) Priority: 19.06.2009 CN 200920014690 U
(71) Applicant: Li, Wenbo, Liaoning 110026 (CN)
(72) Inventor: Li, Wenbo, Liaoning 110026 (CN)
(74) Representative: Dixon, Philip Matthew
(86) International application number: PCT/CN2010/073613
(87) International publication number: WO 2010/145468

(57) **Abstract**

A high-frequency induction atomization device for delivering physiological active substance in atomizing form into a lung for absorption through a respiratory tract includes a housing and an atomizing core (1), a high-frequency generator (6), a sensor (7) and a power supply unit (10) provided in the housing. The power supply unit (10), the sensor (7) and the high-frequency generator (6) are installed in the housing sequentially. The sensor (7) and the high-frequency generator (6) are electrically connected with the power supply unit (10) respectively. The high-frequency generator (6) is provided with an air hole (5), the high-frequency generator (6) is provided with a high frequency coil (3), the atomizing core (1) installed in the housing is inserted into the high frequency coil (3), a gap functioned as a gas flow channel is left between the atomizing core (1) and the high frequency coil (3). The inspiration end of the housing is provided with an air entry (16), and the housing is provided with an inflow port (9). The exothermic electric current of the atomizing core (1), which is stable in performance, is produced by the high-frequency induction. The atomizing core (1) is removable, low in cost, and easy in batch process. The quantitative atomizing is realized by replacing atomizing core (1).

## Description

### TECHNICAL FIELD

The present utility model relates to an atomizing device for lung absorption, particularly to a high frequency induction atomizing device for deliver a vaporized physiological activator through pneogaster to the lungs for absorption.

### BACKGROUND

Presently, a number of different delivery devices for lung absorption have become commercially available, such as ultrasound atomizing delivery devices, or atomizing electronic cigarettes as tobacco substitutes. Ultrasound atomizing delivery devices are relatively large and is inconvenient to carry, in addition, vapor particles generated thereby are too large; atomizers in atomizing electronic cigarettes are DC supply systems which are connected directly or by electronic contact points, which is adverse to usage of disposable atomizers.

### SUMMARY OF THE UTILITY MODEL

To overcome the disadvantage of the arts, one of the objects of the present utility model is to provide a high frequency induction atomizing device operating stably, an atomizing core is powered by an output coil of a high frequency generator via electromagnetic induction in a contactless manner.

Another object of the present utility model is to provide a high frequency induction atomizing device for quantified atomization.

The objects of the present utility model are achieved by the following techniques:

The present utility model comprises a housing and an atomizing core, a high frequency generator, a sensor and a power supply received in the housing, the power supply, sensor and high frequency generator are mounted in the housing in turns, the sensor and the high frequency generator is electrically connected to the power supply, respectively; the high frequency generator is provided with an air vent, a high frequency coil is set in the high frequency generator, the atomizing core mounted in the housing is inserted into the high frequency coil, a gap is formed as an air passage between the atomizing core and the high frequency coil; a suction vent is formed at an inhale end of the housing, an inlet vent is set on the housing.

Wherein: the housing has a holder, one end of the atomizing core is detachably amounted to the holder, the other end of the atomizing core is inserted into the high frequency coil; a liquid storage component is received between the atomizing core and the suction vent in the housing, one end of the atomizing core is connected to the liquid storage component, the other end is inserted into the high frequency coil; an air passage in connection with the air vent and the suction vent is formed between the liquid storage component and an inner wall of the housing; the liquid storage component is made from micropore ceramic, foamed ceramic, natural fibre, artificial fibre or foamed metal materials; atomizing liquid in the liquid storage component or the atomizing core comprises 60∼95% weight percent propylene glycol, 1∼30% weight percent glycerol, and the rest is essence; an annular ferrite is placed outside the high frequency coil, the high frequency coil is amounted to the high frequency generator via the annular ferrite; a closed helix heater is set outside the atomizing core, the atomizing core is made from carbon fibre, stainless steel fibre or foamed metal; an indicator light in connection with the power supply is received between the housing and the power supply; the power supply is a rechargeable or disposable cell; the housing includes a first housing and a second housing, one end of the second housing is detachably coupled to the first housing by plugging, the other end of the second housing is provided with a suction vent; the power supply, the sensor and the high frequency generator are received in the first housing, the high frequency generator is at one side of the sensor, the inlet vent is on the first housing on the other side of the sensor; the sensor includes an air flow sensor or an air pressure sensor; the high frequency generator includes a push-pull output circuit with an operation frequency at 1MHz∼960MHz.

The present utility model has the following advantages and positive effects:

1. In the present utility model, the atomizing core is inserted into the high frequency coil and has no direct electric contact with the high frequency circuit, a current used by the atomizing core for heating is generated by high frequency induction, and hence the atomizing core operates stably.

2. The atomizing core according to the present utility model is disposable and low cost, so it's suitable for volume production.

3. In the present utility model, the liquid storage component can be omitted and the device operated only with the atomizing liquid in the atomizing core, so as to quantify the atomization by changing the atomizing core.

4. In the present utility model, the high frequency coil can be amounted to the high frequency generator by the annular ferrite, the non-conductive annular ferrite acts as an isolation shield to prevent magnet field from escaping.

5. The housing according to the present utility model is assembled by plugging, so as to facilitate changing the atomizing core or the liquid storage component.

### BRIEF DESCRIPTION OF THE DRAWINGS

Fig. 1 is a schematic view illustrating a structure of embodiment 1 of the present utility model.

Fig. 2 is a schematic view illustrating a structure of embodiment 2 of the present utility model.

Wherein, 1 indicates the atomizing core, 2 indicates the helix heater, 3 indicates the high frequency coil, 4 indicates the annular ferrite, 5 indicates the air vent, 6 indicates the high frequency generator, 7 indicates the sensor, 8 indicates the auxiliary inlet vent, 9 indicates the inlet vent, 10 indicates the power supply, 11 indicates the indicator light, 12 indicates the air passage, 13 indicates the first housing, 14 indicates the liquid storage component, 15 indicates the plugging port, 16 indicates the suction vent, 17 indicates the holder, 18 indicates the second housing.

### DETAILED DESCRIPTION OF THE EMBODIMENTS

The present utility model will be further described below with reference to the drawings.

Embodiment 1

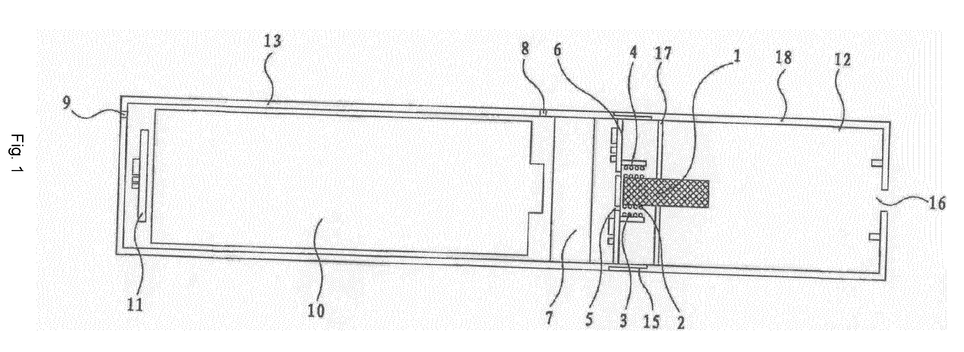

As shown in Fig. 1, the high frequency induction atomizing device in this embodiment comprises a housing and an atomizing core 1, a high frequency generator 6, a sensor 7, a power supply 10 and an indicator light 11 received in the housing. The housing is composed of two parts, a first housing 13 and a second housing 18, the first and second housings 13, 18 are connected to each other by plugging, the second housing 18 is detachable to facilitate changing the atomizing core 1. In Fig. 1, the indicator light 11, the power supply 10, the sensor 7 and the high frequency generator 6 are set in turn in the first housing 13 from left to right, the indicator light 11, the sensor 7 and the high frequency generator 6 are respectively electrically connected to the power supply 10, the power supply can include a rechargeable cell or a disposable cell. One side of the high frequency generator 6 is facing the sensor 7, the other side is provided with the high frequency coil 3, the annular ferrite 4 is set outside the high frequency coil 3 and acts as an isolation shield to prevent magnet field from escaping, the high frequency coil 3 is amounted to the high frequency generator 6 via the annular ferrite 4; a circuit board of the high frequency generator 6 is provided with an air vent 5. The power supply 10 is at the left side of the sensor 7 in Fig. 1, the indicator light 11 is between the power supply 10 and the top inner wall of the first housing 13. The inlet vent 9 is formed on the first housing and located on the left of the sensor 7 in Fig. 1; to further improve the air inflow, the auxiliary inlet vent 8 can be formed on the first housing 13 near the sensor 7. In this embodiment, a holder 17 is amounted in the second housing 18, one end of the atomizing core 1 is detachably set on the holder 17, the other end is inserted into the high frequency coil 3, a gap is formed as an air passage between the atomizing core 1 and the high frequency coil 3. If the atomizing core 1 is made from non-conductive materials, a closed helix heater 2 can be set outside the atomizing core 1, a gap is formed as an air passage between the helix heater 2 and the high frequency coil 3. The suction vent 16 is formed at the tail end of the second housing 18.

The operation mechanism of this embodiment is: when the user put the device in mouth and inhales, the indicator light 11 is lit, air flows into the first housing 13 through the inlet vent 9 and the auxiliary inlet vent 8, the high frequency generator 6 is actuated by the sensor 7, the atomizing core 1 set in the high frequency coil 3 generates a high frequency current by induction, therefore the temperature of the atomizing core 1 raises sharply to the boiling point of the atomizing liquid which is hence vaporized; the inhaled air passes through, in turn, the sensor 7, the air vent 5 on the high frequency generator 6 and the gap between the high frequency coil 3 and the atomizer core 1, the vapor of the atomizing liquid is condensate rapidly to aerosol in the accompanying air flow, and forms simulated smoke at the suction vent 16.

This embodiment can achieve quantified atomization by changing the atomizing core; the atomizing liquid contains 70% weight percent propylene glycol, 25% weight percent glycerol, and the rest is essence.

Embodiment 2

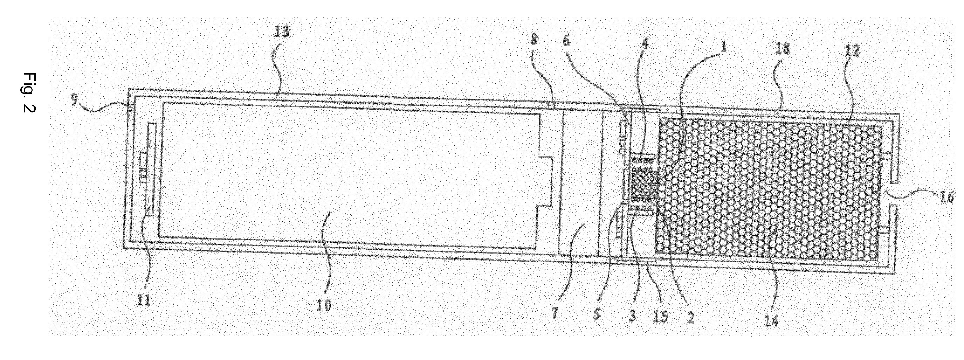

As shown in Fig. 2, this embodiment is different from embodiment 1 in that a liquid storage component 14 is set in the second housing 18, one end of the atomizing core 1 is connected to the liquid storage component 14, the other end is inserted into the high frequency coil 3; an air passage 12 in connection with the air vent 5 and the suction vent 16 is formed between the liquid storage component 14 and the inner wall of the housing.

The operation mechanism of this embodiment is: when the user put the device in mouth and inhales, air flows into the first housing 13 through the inlet vent 9 and the auxiliary inlet vent 8, the high frequency generator 6 is actuated by the sensor 7, the atomizing core 1 set in the high frequency coil 3 generates a high frequency current by induction, therefore the temperature of the atomizing core 1 raises sharply to the boiling point of the atomizing liquid in the atomizing core 1 and the liquid storage component 14, the atomizing liquid is hence vaporized; the inhaled air passes through, in turn, the sensor 7, the air vent 5 on the high frequency generator 6 and the gap between the high frequency coil 3 and the atomizer core 1, the vapor of the atomizing liquid is condensate rapidly to aerosol in the accompanying air flow, and forms simulated smoke at the suction vent 16. The atomizing liquid contains 80% weight percent propylene glycol, 15% weight percent glycerol, and the rest is essence.

The atomizing core of this utility model can be made from carbon fibre, stainless steel fibre, foamed metal; the high frequency generator 6 can include a push-pull output circuit with an operation frequency at 1MHz ∼ 960MHz; the sensor 7 can include an air flow sensor or an air pressure sensor; the liquid storage component 14 can be made from micropore ceramic, foamed ceramic, natural fibre, artificial fibre or foamed metal materials; the atomizing liquid comprises 60∼95% weight percent propylene glycol, 1∼30% weight percent glycerol, and the rest is essence.

## Claims

1. A high frequency induction atomizing device, **characterized in**: comprising a housing and an atomizing core (1), a high frequency generator (6), a sensor (7) and a power supply (10); the power supply (10), the sensor (7) and the high frequency generator (6) is mounted in turn in the housing; the sensor (7) and the high frequency generator (6) are electrically connected to the power supply (10), respectively; the high frequency generator (6) is provided with an air vent (5), a high frequency coil (3) is provided on the high frequency generator (6); the atomizing core (1) mounted in the housing is inserted into the high frequency coil (3); a gap is formed as an air passage between the atomizing core (1) and the high frequency coil (3); an suction vent (16) is formed at a suction end of the housing, an inlet vent (9) is formed on the housing.

2. The high frequency induction atomizing device of claim 1, **characterized in that**: the housing has a holder (17), one end of the atomizing core (1) is detachably mounted to the holder (17), and the other end is inserted into the high frequency coil (3).

3. The high frequency induction atomizing device of claim 1, **characterized in that**: the housing further includes a liquid storage component (14) between the atomizing core (1) and the suction vent (16); one end of the atomizing core (1) is connected to the liquid storage component (14); the other end is inserted into the high frequency coil (3); an air passage (12) in connection with the air vent (5) and the suction vent (16) is formed between the liquid storage component (14) and an inner wall of the housing.

4. The high frequency induction atomizing device of claim 3, **characterized in that**: the liquid storage component (14) is made from micropore ceramic, foamed ceramic, natural fibre, artificial fibre or foamed metal materials.

5. The high frequency induction atomizing device of claim 3, **characterized in that**: an atomizing liquid in the liquid storage component (14) or the atomizing core (1) is constituted of, by weight percentages, 60-95 % of propylene glycol, 1-30% of glycerol, and flavoring essence at a remaining percentage.

6. The high frequency induction atomizing device of claim 1 or 3, **characterized in that**: an annular ferrite (4) is placed outside the high frequency coil (3), and the high frequency coil (3) is amounted to the high frequency generator (6) via the annular ferrite (4).

7. The high frequency induction atomizing device of claim 1 or 3, **characterized in that**: a closed helix heater (2) is set outside the atomizing core (1), and the atomizing core (1) is made from carbon fibre, stainless steel fibre or foamed metal.

8. The high frequency induction atomizing device of claim 1 or 3, **characterized in that**: an indicator light (11) electrically connected with the power supply (10) is positioned between the housing and the power supply (10); the power supply (10) is a rechargeable or disposable cell.

9. The high frequency induction atomizing device of claim 1 or 3, **characterized in that**: the housing includes a first housing (13) and a second housing (18); one end of the second housing (18) is detachably coupled to the first housing (13) by plugging; the other end of the second housing (18) is provided with the suction vent (16); the power supply (10), the sensor (7) and the high frequency generator (6) are positioned in the first housing (13); the high frequency generator (6) is at one side of the sensor (7), the inlet vent (9) is formed on the first housing (13) and on the other side of the sensor (7); the sensor (7) includes an air flow sensor or an air pressure sensor.

10. The high frequency induction atomizing device of claim 1 or 3, **characterized in that**: the high frequency generator (6) includes a push-pull output circuit with an operation frequency at 1 MHz-960MHz.
